# EUROPÄISCHE PATENTANMELDUNG

(11) **EP 4 105 706 A1**
(43) Veröffentlichungstag der Anmeldung: **21.12.2022**
(21) Anmeldenummer: 21195045.6
(22) Anmeldetag: 06.09.2021
(51) Int. Cl.: G02B 21/34, G01N 23/225, G02B 21/36

(54) **VERFAHREN ZUR KORRELATIVEN MIKROSKOPIE**

(30) Priorität: 17.06.2021 EP 21180173
(71) Anmelder: Leica Mikrosysteme GmbH, 1170 Wien (AT)
(72) Erfinder: DR. WURZINGER, Paul, 2232 Deutsch-Wagram (AT); DR. KÖNIG, Julia, 1070 Wien (AT)
(74) Vertreter: DehnsGermany Partnerschaft von Patentanwälten

(57) **Zusammenfassung**

Es wird ein Verfahren (400) zur mikroskopischen Untersuchung einer Probe (1) vorgeschlagen, das die Schritte (a) Aufbringen (410) der Probe (1) auf einen Probenträger (10) mit oder bestehend aus einem lichtdurchlässigen Trägermaterial, (b) Aufnehmen (420) eines ersten Bildes (210, 220) der auf dem Probenträger (10) aufgebrachten Probe (1) mit einem ersten lichtmikroskopischen Verfahren, wobei das erste Bild (210, 220) durch erste Bilddaten repräsentiert wird, (c) Kryofixieren, Gefriersubstituieren und anschließendes Infiltrieren und Einbetten (430) der Probe (1) zusammen mit dem Probenträger (10) mit einem Einbettmedium (20) in einer Einbettform (90, 100), (d) Aushärten (440) des Einbettmediums (20), (e) Entnahme der im ausgehärteten Einbettmedium (20) eingebetteten Probe (1) zusammen mit dem Einbettmedium (20) und dem Probenträger (10) aus der Einbettform (90, 100), (f) Aufnehmen (450) eines zweiten Bildes (230) der im ausgehärtetem Einbettmedium (20) eingebetteten Probe (1) mit einem zweiten lichtmikroskopischen Verfahren, wobei das zweite Bild durch zweite Bilddaten repräsentiert wird und durch das erste und das zweite Aufnehmen zumindest teilweise identische Bereiche der Probe (1) aufgenommen werden und (g) Identifizieren (460) von zumindest eines Ausschnittes des ersten Bildes (210, 220) und eines Ausschnittes des zweiten Bildes (230), die identische Bereiche der Probe (1) zeigen, umfasst.

## Beschreibung

Die vorliegende Erfindung bezieht sich auf die Gebiete der Lichtmikroskopie und der korrelativen Mikroskopie.

### Hintergrund der Erfindung

Die Erfindung betrifft ein Verfahren zur Zuordnung von in lichtmikroskopischen Aufnahmen abgebildeten Bereichen einer biologischen Probe zu den gleichen Probenbereichen nach Hochdruckgefrieren und Gefriersubstitution sowie einer Kryosubstitution und Einbettung in ein Einbettmedium.

Die korrelative Licht- und Elektronenmikroskopie (Correlative Light and Electron Microscopy, CLEM) ist bekannt und beispielsweise bei Gibson, K. H. et al., Fluorescing the Electron: Strategies in Correlative Experimental Design, Kapitel 2 in: Müller-Reichert, T. und Verkade, P. (Hrsg.), Correlative Light and Electron Microscopy II, Methods in Cell Biology, Bd. 124, Elsevier/Academic Press 2014, beschrieben. Bei der korrelativen Licht- und Elektronenmikroskopie werden Proben zunächst mittels Licht- oder Fluoreszenzmikroskopie untersucht, dann für die Elektronenmikroskopie vorbereitet, und schließlich mittels Elektronenmikroskopie analysiert, wobei mittels der Licht- und der Elektronenmikroskopie erhaltene Bilddaten miteinander korreliert werden.

Biologische Proben in Form von Zellkolonien, Zellkulturen, Biofilmen und dergleichen werden häufig auf transparente Materialien aufgewachsen bzw. auf diesen gezüchtet, die somit als Trägermaterialien bzw. Probenträger für diese Proben agieren. Präparate dieser Proben sollen in der korrelativen Licht- und Elektronenmikroskopie zunächst der lichtmikroskopischen und anschließend der elektronenmikroskopischen Untersuchung zugänglich gemacht werden. Hierbei sollen bestimmte Probenbereiche, wie beispielsweise bestimmte Zellen, Zellstrukturen u. ä., untersucht werden.

Hierfür werden die auf einen Probenträger (z.B. bestehend aus einem transparenten Trägermaterial wie Quarz) aufgebrachten bzw. auf diesem kultivierten Proben typischerweise zuerst lichtmikroskopisch untersucht, zum Beispiel mittels Durchlichtmikroskopie. Die auf dem Probenträger befindlichen Proben werden anschließend kryofixiert, gefriersubstituiert, und mittels eines Einbettmediums wie Kunstharz infiltriert und gleichzeitig in dieses eingebettet. Anschließend wird dieses ausgehärtet. Dabei befindet sich die Probe (bspw. die zu untersuchenden Zellen) fortwährend in Kontakt mit dem Probenträger. Durch die Einbettung werden Präparatblöcke erhalten, von denen mittels eines Ultramikrotoms Ultradünnschnitte hergestellt werden können.

Mitunter ist es in der korrelativen Licht- und Elektronenmikroskopie von Interesse, vor der Fixierung auf dem Probenträger identifizierte Bereiche von Interesse der Probe, beispielsweise bestimmte Zellaggregate oder Zellen, die eine bestimmte physiologische Reaktion oder Eigenschaft zeigen, nach der Fixierung nochmals aufzufinden bzw. vergleichend lichtmikroskopisch zu untersuchen. Die Erfindung stellt sich insbesondere die Aufgabe, ein Verfahren anzugeben, das dies in vorteilhafter Weise ermöglicht.

### Offenbarung der Erfindung

Vor diesem Hintergrund schlägt die Erfindung ein Verfahren mit den Merkmalen des Hauptanspruchs vor. Ausgestaltung der Erfindung sind Gegenstand der abhängigen Ansprüche und der nachfolgenden Beschreibung.

Vor der weiteren Erläuterung der erfindungsgemäß vorgeschlagenen Maßnahmen werden zunächst im Rahmen der vorliegenden Erfindung anwendbare Techniken erläutert und zur Erläuterung der Erfindung verwendete Begriffe definiert.

Die Kryofixierung ist ein für die Elektronenmikroskopie häufig eingesetztes Probenpräparationsverfahren. Dabei wird eine wasserhaltige Probe sehr rasch auf eine Temperatur von unter -150 °C abgekühlt und erstarrt dabei im Wesentlichen ohne die Bildung von Eiskristallen. Die Kryofixierung erfolgt beispielsweise mittels bzw. in Form von Hochdruckgefrieren (siehe unten). Die Kryofixierung hat sich als besonders geeignet für strukturbiologische Untersuchungen erwiesen. Die zu untersuchenden Objekte, beispielsweise Zellen, Enzyme, Viren oder Lipidschichten, werden dadurch in einer verifizierten Eisschicht eingebettet. Der große Vorteil der Kryofixierung liegt darin, dass die biologischen Strukturen in ihrem nativen Zustand erhalten werden können. Unter anderem kann ein biologischer Vorgang zu einem beliebigen Zeitpunkt durch Kryofixierung angehalten und in diesem verifizierten Zustand untersucht werden, z.B. im Kryo-Elektronenmikroskop aber auch in einem Lichtmikroskop mit entsprechender Probenkühlung, wobei die Kryo-Lichtmikroskopie vor allem zum Auffinden von relevanten Bereichen in der Probe dient, welche Bereiche vorgemerkt und anschließend in einem Kryo-Elektronenmikroskop genauer betrachtet werden. Ein geeignetes Lichtmikroskop hierzu ist beispielsweise aus der WO 2016/016000 A1 bekannt.

Unter Hochdruckgefrieren versteht man das Gefrieren von Proben unter Hochdruck. Es handelt sich um ein Verfahren zur Kryofixierung. Durch das Gefrieren bei Drücken von in etwa 2000 bar werden bspw. Eiskristallbildung unterdrückt und strukturelle Schäden an der Probe vermieden, weil Wasser bei entsprechenden Drücken direkt vom flüssigen in den vitrifizierten Zustand übergeht.

Gefriersubstitution ist ein häufig nach dem Hochdruckgefrieren und anderen Kryofixiermethoden angewendetes Verfahren. Dabei wird der gefrorenen Probe mithilfe eines organischen Lösungsmittels bei niedrigen Temperaturen und häufig in Gegenwart eines sekundären Fixiermittels das Wasser entzogen. Anschließend wird das Lösungsmittel durch ein aushärtbares bzw. polymerisierbares Material, z.B. ein Kunstharz, ersetzt. Ist die Gefriersubstitution abgeschlossen, erfolgt die Aushärtung typischerweise durch Polymerisation des Kunstharzes wie beispielsweise Epoxidharz.

Nach der Aushärtung wird der Probenträger abgelöst. (Im Folgenden sei unter dem Begriff Präparatblock der ausgehärtete Block bestehend aus dem ausgehärteten Einbettmedium einschließlich der eingebetteten Probe zu verstehen.) Probenbereiche, die im Lichtmikroskop interessierende Reaktionen oder Zellzustände zeigten, werden dann für eine elektronenmikroskopische Untersuchung weiter präpariert. Das Ziel ist es, bei der elektronenmikroskopischen Bildaufnahme gezielt interessierende Probenbereiche aufnehmen zu können, die vorher lichtmikroskopisch identifiziert worden sind. Zur Unterstützung der Korrelation zwischen lichtmikroskopischem und elektronenmikroskopischem Bild ist der Probenträger meist strukturiert oder mit einer lokal eindeutig unterscheidbaren Gitter- oder Netzstruktur überzogen/beschichtet.

Die Präparate werden häufig in sogenannten Kryoröhrchen (auch als Cryovials oder auch Microtubes bezeichnet) verschiedener Hersteller eingebettet und ausgehärtet, die typischerweise im oberen Bereich zylindrisch ausgebildet sind und nach unten konisch zulaufen. Hierbei sinkt der Probenträger mit der Probe im Einbettmedium im Kryoröhrchen soweit ab, dass er vollumfänglich durch das Gefäß umschlossen wird und den entstehenden Präparatblock in zwei Teile unterteilt. So gestaltete Kryoröhrchen führen auch oft dazu, dass die Probenträger im unteren, konischen Bereich schräg zu liegen kommen und damit die Probe nicht definiert ausgerichtet ist. Außerdem ist die Methode für gebrochene Probenträger weitgehend unbrauchbar, weil die Bruchstücke bei solchen Kryoröhrchen voll vom ausgehärteten Einbettmedium (dem Präparatblock) umgeben und kaum aus dem Präparatblock zu trennen sind.

Von der Anmelderin werden deshalb sogenannte Flow-Through-Ringe ("flow-through rings") oder auch Flow-Through-Einsätze ("flow-through inserts"; für den Einsatz in eine umgebende Wanne) angeboten, in denen der Probenträger vollflächig auf einer berandenden Struktur (nämlich dem Boden eines im Wesentlichen zylindrischen Ringabschnitts) aufliegt. Dadurch befindet sich der ausgehärtete Block auf der mit der Probe belegten Seite des Trägermaterials, während sich auf der Rückseite nur ein dünner Polymerfilm zwischen dem Formboden und dem Trägermaterial bildet. Der Boden ist den Abmessungen der Probenträger angepasst. Hierdurch wird eine nachteilhafte Verkantung/Schrägstellung des Probenträgers vermieden. Die Verwendung entsprechender Ringe bzw. Einsätze ist fachüblich und beispielsweise in der eingangs erwähnten Publikation von Gibson et al. beschrieben. Entsprechende Gefäße weisen, wie bereits zuvor mit anderen Worten ausgedrückt, beispielsweise zumindest einen zylindrischen Abschnitt auf, dessen Innendurchmesser derart bemessen ist, dass in ihn der Probenträger einsetzbar ist, wobei der zylindrische Abschnitt an einem Axialende eine nach innen ragende Struktur aufweist, die zum Abstützen des Probenträgers eingerichtet ist.

Die Grenzfläche zwischen dem ausgehärteten Präparatblock und dem Probenträger wird während des Ablösens des Probenträgers oft beschädigt oder uneben. Die häufig mittels Beschichtung aufgebrachte Gitter- oder Netzstruktur auf dem Trägermaterial, an der man sich in der lichtmikroskopischen Untersuchung orientieren konnte, löst sich oft (lokal) mit dem Trägermaterial des Probenträgers ab und damit wird die Zuordnung zwischen lebenden und gefriersubstuierten und ausgehärteten Präparatteilen schwierig und unpräzise. Lokale Beschädigungen der Blockoberfläche (Oberfläche des Blocks bestehend aus dem Einbettmedium, in dem die Probe eingebettet wurde, beispielsweise ein Kunstharzblock) führen zu lokalen Lücken in der Auswertung und können auch für unbeschädigte Präparatteile eine ernste Beeinträchtigung der Zuordnung bewirken.

Die vorliegende Erfindung schafft vor diesem Hintergrund ein einfach zu implementierendes und kostengünstiges Verfahren, das eine einfache und fehlertolerante Identifizierung und Zuordnung von sich entsprechenden Bildbereichen von Bildern der ursprünglichen Probe und Aufnahmen in späteren Untersuchungsschritten wie beispielsweise elektronenmikroskopischen Aufnahmen im Rahmen der erwähnten korrelativen Licht- und Elektronenmikroskopie ermöglicht.

Gemäß einem ersten Aspekt der Erfindung umfasst ein erfindungsgemäßes Verfahren zur mikroskopischen Untersuchung einer Probe die gemäß Hauptanspruch vorgesehenen und nachfolgend weiter erläuterten Schritte:
- Aufbringen der Probe auf einen Probenträger bestehend mit oder aus einem lichtdurchlässigen Trägermaterial,
- Aufnehmen eines ersten Bildes der auf dem Probenträger aufgebrachten Probe mit einem ersten lichtmikroskopischen Verfahren (im Fall der Untersuchung lebender Zellen insbesondere einem lichtmikroskopischen Verfahren, das für die Lebendzellmikroskopie geeignet ist),
- Kryofixieren, Gefriersubstituieren und anschließendes Infiltrieren und Einbetten der Probe mit einem Einbettmedium, beispielsweise einem Kunstharz, in einer Einbettform,
- Aushärten, beispielsweise Polymerisieren, des Einbettmediums,
- Entnahme der im ausgehärteten Einbettmedium eingebetteten Probe zusammen mit dem Einbettmedium und dem Probenträger aus der Einbettform,
- rein optional: Ablösen (Entfernen) des ausgehärteten Einbettmediums von der Rückseite (der Seite des Probenträgers, die der Probe abgewandt ist) des Probenträgers, wobei dieses Reinigen bevorzugt zumindest teilweise mechanisch erfolgt, aber auch durch chemische Reinigungsmittel wie Ethanol unterstützt werden kann,
- Aufnehmen eines zweiten Bildes der im ausgehärteten Einbettmedium eingebetteten Probe mit dem ersten oder einem zweiten lichtmikroskopischen Verfahren, während der Probenträger an dem der Probe zugewandten Seite an dem ausgehärteten Einbettmedium bzw. zumindest einen Teil hiervon, der zuvor nicht entfernt wurde, haftet, insbesondere durch den Probenträger hindurch, wobei das zweite Bild durch zweite Bilddaten repräsentiert wird und durch das erste und das zweite Aufnehmen zumindest teilweise identische Bereiche der Probe aufgenommen werden, und schließlich
- Identifizieren mindestens eines Ausschnittes des ersten Bildes und mindestens eines Ausschnittes des zweiten Bildes, die identische Bereiche der Probe zeigen.

Im Rahmen dieser Anmeldung ist "Aufnehmen eines Bildes" verallgemeinert zu verstehen und umfasst auch die Aufnahme von z.B. Bildfolgen wie zeitliche/räumliche Bildserien und Fokus-Serien oder auch Kombinationen hiervon. Beispiele für lichtmikroskopische Verfahren, die als das erste und/oder das zweite lichtmikroskopische Verfahren eingesetzt werden können, sind Formen der Lichtmikroskopie wie Durchlichtmikroskopie, Auflichtmikroskopie, Fluoreszenzmikroskopie etc. und auch Kombinationen davon. Hierbei können sowohl scanmikroskopische als auch flächendetektorbasierte ("Widefield") Mikroskopieverfahren zum Einsatz kommen. Auch ist es vorstellbar, dass unterschiedliche Teilbilder eines Bildes mit unterschiedlichen Formen der Lichtmikroskopie aufgenommen werden, auch ein solches kombiniertes Aufnahmeverfahren ist im Rahmen der Anmeldung ein lichtmikroskopisches Verfahren. Ein aufgenommenes Bild wird jeweils durch die aufgenommenen Bilddaten repräsentiert.

Als Ausgangsmaterial für das erfindungsgemäße Verfahren werden Proben wie beispielsweise Zellen oder Gewebeteile auf einen Probenträger aufgebracht, also beispielsweise aufgewachsen und/oder aufgenommen, und mit einem Lichtmikroskop abgebildet. Da solche Proben bis zum Kryofixieren häufig noch lebend sind (also ein Lebendpräparat darstellen), ist es besonders vorteilhaft, wenn die abzubildende Probe bis und/oder während der Aufnahme des ersten Bildes unter kontrollierten, d.h. insbesondere physiologisch günstigen oder eine bestimmte physiologische Reaktion hervorrufenden Bedingungen gehalten wird. Unter physiologisch günstigen Bedingungen sind Umgebungsbedingungen zu verstehen, die für eine zu untersuchende lebende Probe wie eine Zelle oder mehrere Zellen nicht schädlich sind und insbesondere ungestörte physiologische Reaktionen sicherstellen. Beispielsweise kann die Probe hierfür zusammen mit einem Probenträger in einer Kapsel untergebracht sein, in der eine bestimmte Temperatur und/oder Zusammensetzung der Luft (beispielsweise ein bestimmter Sauerstoffgehalt) sichergestellt sind.

Unter einem Bild einer Probe im Sinne der vorliegenden Anmeldung ist eine zumindest teilweise Abbildung der Probe zu verstehen. Ein Bild einer Probe kann also die gesamte Probe zeigen oder auch nur Teilbereiche. Das erfindungsgemäße Verfahren ist also nicht auf Bilder der gesamten Probe beschränkt. Unter den Begriff eines Bildes sollen im Rahmen dieser Anmeldung auch räumliche oder zeitliche Folgen aus Einzelbildern (Bildserien) fallen, solange zumindest eines dieser Teilbilder eine teilweise Abbildung der Probe enthält (also einen Teilbereich der Probe darstellt).

Als lichtmikroskopisches Verfahren zur Aufnahme sowohl des ersten als auch des zweiten Bildes kommt insbesondere die Durchlicht-Hellfeldmikroskopie in Frage, allerdings ist, wie oben erwähnt, eine große Breite an lichtmikroskopischen Verfahren wie z.B. Auflichtmikroskopie, Fluoreszenzmikroskopie, Konfokalmikroskopie oder Multiphotonenmikroskopie denkbar. Auch sind sowohl scannende als auch kamerabasierte Verfahren (Widefield-Mikroskope) denkbar.

Des Weiteren kann ein Bild der Probe sich auch aus mehreren Teilaufnahmen (Einzelbildern) zusammensetzen. Diese Teilaufnahmen werden dann für die weiteren Verfahrensschritte zu einer großflächigen Gesamtaufnahme vereinigt (Stitching).

Gemäß einem weiteren Aspekt der Erfindung erfolgt das Aufnehmen des ersten Bildes und das Aufnehmen des zweiten Bildes vorzugsweise unter Verwendung desselben Mikroskops oder eines Mikroskops desselben Typs. Hierdurch wird eine einfache Vergleichbarkeit zwischen erstem und zweitem Bild sichergestellt.

Nach einem weiteren erfindungsgemäßen Aspekt erfolgt das Aufnehmen des ersten Bildes und das Aufnehmen des zweiten Bildes unter Verwendung desselben Objektivs und/oder bei derselben Vergrößerung und/oder unter Verwendung desselben Scanbereichs und/oder derselben Bildauflösung. Das Ziel ist es auch hier, möglichst vergleichbare Bedingungen für die Bildaufnahme sicherzustellen, um eine gute Vergleichbarkeit des ersten und des zweiten Bildes zu gewährleisten. Insbesondere ist es auch vorteilhaft, wenn das Aufnehmen des ersten Bildes und das Aufnehmen des zweiten Bildes von der gleichen Probenseite erfolgt, also z.B. durch das transparente Trägermaterial bzw. den Probenträger. Damit ergibt sich auch ein Vorteil der oben angeführten Verwendung desselben Objektivs, da dieses Objektiv vorteilhafterweise auf die Abbildung durch den verwendeten Probenträger korrigiert ist.

Nach Durchführung der oben beschriebenen Schritte erfolgt vor der Weiterverarbeitung des ausgehärteten Präparates typischerweise ein zumindest teilweises Ablösen des Probenträgers von dem Präparat (also der im Einbettmedium eingebetteten Probe) nach Aufnahme des zweiten Bildes.

Erfindungsgemäß werden die auf einen Probenträger aufgebrachten Proben zumindest für die Aufnahme des zweiten Bildes vorzugsweise durch das Trägermaterial (das Material des Probenträgers) hindurch untersucht. Die Aufnahme des zweiten Bildes erfolgt also vorzugsweise durch den Probenträger hindurch. Die Probe wird hierzu typischerweise von der der Probe abgewandten Seite (der Rückseite) des Probenträgers durch ein einzelnes Objektiv beleuchtet und auch abgebildet. Daher muss das Trägermaterial ein zumindest teilweise lichtdurchlässiges Material, z.B. Glas oder Saphir, sein. Für einen Probenträger ist also beispielsweise ein Deckglas oder ein in optischer Qualität poliertes Saphirplättchen geeignet. Bei einer bevorzugten Version des erfindungsgemäßen Verfahrens wird folglich als Trägermaterial ein Glas oder Saphir verwendet.

Um eine hohe Qualität der Aufnahme sicherzustellen, ist es vorteilhaft, im Fall einer Aufnahme des zweiten Bildes durch den Probenträger das Einbettmedium von der der Proben abgewandten Seite (der Rückseite) zu entfernen, um eine gute optische Qualität der Abbildung sicherzustellen. Saphir ist hier besonders vorteilhaft, da er auf Grund seiner Härte verhältnismäßig einfach vom Einbettmedium mechanisch getrennt werden kann: Es können im Fall von Saphir metallische und weiche abrasive Materialien verwendet werden, um das Einbettmedium vom Probenträger zu entfernen, ohne die Oberfläche des Probenträgers zu zerkratzen. Das Einbettmedium kann somit restlos oder zumindest weitestgehend entfernt werden, so dass die Oberfläche des Probenträgers wieder eine ausreichende optische Qualität erlangt und eine möglichst optimale Abbildung bzw. Aufnahme des zweiten Bildes zulässt.

Als Einbettmedium kommt erfindungsgemäß bevorzugt ein Kunstharz, insbesondere ein Epoxidharz zum Einsatz.

Ein abschließendes Loslösen des Probenträges vom Präparatblock zum Zweck der Weiterverarbeitung kann beispielsweise durch Eintauchen in LN₂ (Flüssigstickstoff) geschehen, wobei die unterschiedliche Wärmeausdehnungskoeffizienten von Einbettmedium und Probenträger zum Abspringen des Probenträgers führen. Alternativ oder zusätzlich ist auch ein Ablösen des Probenträgers mit einer Klinge wie einer Skalpellklinge möglich.

Eine besonders bevorzugte Variante des erfindungsgemäßen Verfahrens nutzt ein Einbettmedium, das Autofluoreszenz aufweist, also zur Autofluoreszenz fähig ist, beispielsweise ein Kunstharz. Diese Autofluoreszenz wird ausgenutzt, um die zweite lichtmikroskopische Aufnahme durchzuführen: Zur Bildaufnahme kann das Einbettmedium zur Autofluoreszenz, beispielsweise mittels einer externen Lichtquelle wie einer LED oder einem Laser, angeregt werden. Das fluoreszierende Licht bestrahlt dann wiederum die Probe und dient damit als Durchlichtquelle zur Aufnahme des zweiten lichtmikroskopisches Bildes. Das zweite lichtmikroskopische Bild wird also durch Durchlicht-Hellfeldmikroskopie aufgenommen. Hierdurch ist es auf besonders einfache Weise möglich, eine mikroskopische Aufnahme des Präparates anzufertigen, ohne dieses besonders aufbereiten zu müssen.

So ist eine glatte Oberfläche des Präparatblocks an der Eintrittsstelle des Lichts für einen möglichst verlustfreien Eintritt zwar vorteilhaft, aber nicht zwingend. Ein Vorteil bei dieser Variante ist es auch, dass (im Gegensatz zu einer direkten Durchlichtbeleuchtung der Probe) durch die Entstehung des Lichts zur Durchlichtbeleuchtung nahe der Probe eine gewisse Absorption des eingestrahlten Lichts akzeptabel ist: Das Verfahren kann trotz mangelhafter Transparenz des Einbettmediums für die Wellenlänge des Lichts der externen Lichtquelle und/oder die Wellenlänge der Autofluoreszenz durchgeführt werden. Weiterhin ist bei dieser Variante eine Beleuchtung des Präparatblocks aus einer beliebigen Richtung möglich, bspw. können Beleuchtung und Detektion durch entgegengesetzte Seiten des Präratblocks erfolgen oder die Richtung der Beleuchtung kann senkrecht zur Richtung der Detektion erfolgen. Eine gleichzeitige oder zeitversetzte Beleuchtung aus mehreren Richtungen ist natürlich ebenfalls denkbar, beispielsweise mittels mehrerer künstlicher Lichtquellen, also beispielsweise mehrerer LEDs und/oder Laser, oder auch mehrerer Teilstrahlen, die durch Aufspaltung des Lichts einer einzelnen Lichtquelle erzeugt werden. Häufig ist ein senkrechter Einfall des Lichts auf eine Oberfläche des Präparatblocks zu bevorzugen, um Reflexionen zu vermeiden, wobei aber auch andere Einfallrichtungen denkbar sind.

Besonders vorteilhaft ist es für dieses Verfahren, wenn die Beleuchtung mittels einer künstlichen Lichtquelle durch dasselbe Objektiv eines Mikroskops erfolgt, das auch für die Abbildung der Probe, also beim zweiten lichtmikroskopischen Verfahren verwendet wird. Anders ausgedrückt wird das Mikroskop sowohl für Beleuchtung und Detektion eingesetzt, und Beleuchtung und Detektion erfolgen durch ein- und dasselbe Objektiv.

Eine aufwendigere Alternative zur Erreichung einer besonders guten Durchlichtbeleuchtung (ohne oder zusätzlich zur Ausnutzung einer Autofluoreszenz des Einbettmediums) ist es, den Präparatblock (ausgehärteter Block einschließlich der Probe) so abzuschneiden und anschließend zu polieren (glätten), dass eine möglichst glatte Oberfläche entsteht und ein verlustarmes (also möglichst reflexions- und streuungsfreies) Eintreten von Licht einer externen Lichtquelle in den Präparatblock ermöglicht wird. Erfolgt ein solches Polieren nicht, ist man davon abhängig, dass die Beleuchtung zufällig an der interessierenden Stelle gut (also verlustarm) eintreten kann, was durch die Meniskusbildung des Kunstharzes an den Wänden der Polymerisationsform typischerweise nicht gegeben ist. Auch in diesem Fall erfolgt die Detektion wieder bevorzugt durch den Probenträger. Ein solches Polieren der Eintrittsstelle ist natürlich auch im Falle der Ausnutzung einer Autofluoreszenz des Einbettmediums vorteilhaft, um die Verluste bei Eintreten des Lichts in den Präparatblock zu minimieren und damit die Autofluoreszenz zu maximieren.

Alternativ oder zusätzlich zur Ausnutzung einer eventuell vorhandenen Autofluoreszenz des Einbettungsmediums ist es auch möglich, dass eine Probe verwendet wird, die zur Fluoreszenz fähig ist, und das zweite lichtmikroskopische Verfahren die Fluoreszenz der Probe detektiert. In diesem Fall handelt es sich bei dem zweiten lichtmikroskopischen Verfahren folglich um ein fluoreszenzmikroskopisches Verfahren. Auch in diesem Fall kann die Beleuchtung (Anregung) der Probe und die Detektion des Fluoreszenzlichtes durch ein- und dasselbe Objektiv eines Mikroskopes erfolgen. Falls die Beleuchtung nicht durch den Probenträger erfolgt, ist auch im Fall der Anregung einer Fluoreszenz der Probe ein Polieren der Eintrittsstelle des Lichts wie oben beschrieben vorteilhaft.

Um eine einfache Weiterverarbeitung und gute Beleuchtung bzw. Detektion durch den Probenträger sicherzustellen, erfolgt das erfindungsgemäße Verfahren vorzugsweise unter Verwendung eines geeigneten Kryoröhrchens mit einem ausreichend breiten, planaren Boden: Der Probenträger mit der Probe wird also vorzugsweise in ein Kryoröhrchen mit flachem Boden eingebracht, wobei die maximale Ausdehnung des Bodens größer oder gleich ist als die maximale Ausdehnung des Probenträgers. Anschließend erfolgt Zuführen des Einbettmediums in das Kryoröhrchen mit der Probe, mindestens so lange bis die Probe vollständig vom Einbettmedium umschlossen ist, wobei der Probenträger auch nach Zuführung des Einbettmediums parallel oder im Wesentlichen parallel zum Boden des Kryoröhrchens orientiert ist. Geeignete Kryoröhrchen für ein solches Verfahren sind beispielsweise die erwähnten Flow-Through-Ringe/Einsätze.

Nach einem weiteren erfindungsgemäßen Aspekt erfolgt die Identifizierung sich entsprechender Probenbereiche auf Basis der ersten Bilddaten und der zweiten Bilddaten mittels eines ersten Algorithmus, beispielsweise mittels bilderkennender Verfahren. Ein solcher Algorithmus kann zumindest teilweise maschinenbasierte Verfahren (Machine Learning/Deep Learning) einsetzen, um ähnliche Strukturen in beiden Bildern bzw. den entsprechenden Bilddaten zu identifizieren. Die Identifizierung sich entsprechender Probenbereiche erfolgt typischerweise durch Analyse der Probenstruktur selbst, kann aber auch durch eine auf dem Probenträger aufgebrachte (beispielsweise durch Beschichtung) künstliche Gitter- oder Netzstruktur unterstützt werden. Alternativ oder zusätzlich ist es natürlich auch denkbar, dass eine Identifizierung sich entsprechender oder Bildbereiche/- strukturen in den Bildern nicht durch einen Algorithmus automatisiert erfolgt, sondern auch manuell (also durch einen Nutzer einer entsprechenden Bildverarbeitungssoftware) durchgeführt wird.

Vorzugsweise wird dem ersten Bild ein erstes Koordinatensystem und dem zweiten Bild ein zweites Koordinatensystem zugeordnet und ausgehend von den miteinander identifizierten Bildbereichen eine Koordinatentransformation zwischen dem ersten Koordinatensystem und dem zweiten Koordinatensystem durch den ersten Algorithmus oder auch einem getrennten zweiten Algorithmus bestimmt. Hierzu sei betont, dass der Einsatz eines Algorithmus (also einer Software) zur Bestimmung einer Koordinatentransformation unabhängig davon ist, ob zur Identifizierung sich entsprechender Probenbereiche bereits ein Algorithmus eingesetzt wurde. Die Koordinatentransformation kann dabei abschnittsweise für lokale Bereiche (bereichsbezogen), wie beispielsweise für spezielle Probenstrukturen, oder auch stetig für das gesamte Präparat bestimmt werden, je nach Aufgabenstellung. Für unterschiedliche Probenbereiche und/oder unterschiedliche Zelltypen innerhalb der Probe können somit unterschiedliche Koordinatentransformationen festgelegt werden. Eine nur für ausgesuchte lokale Bereiche bestimmte Koordinatentransformation ist beispielsweise dann vorteilhaft, wenn sich bestimmte Teile der Probe (wie einzelne Zellen) nach der Aufnahme des ersten Bildes und vor der Aufnahme des zweiten Bildes verschoben (delokalisiert) haben. In diesem Fall ist es vorteilhaft, die Koordinatentransformation nur auf Basis der Teile der Probe zu bestimmen, deren Lage sich nicht geändert hat (also beispielsweise festsitzende Zellen). Einem erfahrenen Nutzer und dementsprechend auch einem entsprechend trainierten neuronalen Netzwerk ist es möglich, bspw. durch Ausschließungsverfahren, delokalisierte Teilbereiche zu identifizieren und nur nicht-delokalisierte Teilbereiche zur Bestimmung der Koordinatentransformation auszuwählen. Sobald eine Koordinatentransformation bestimmt worden ist, lassen sich auf einfache Weise diejenigen Teilbereiche der Probe im ersten Bild bestimmen, die delokalisiert worden sind, da dies alle sind, bei denen die Koordinatentransformation im zweiten Bild kein entsprechendes Gegenstück finden kann. Wiederum durch Ausschließungsverfahren o.ä. lassen sich diese delokalisierten Bereiche typischerweise trotzdem im zweiten Bild identifizieren.

Das zweite Koordinatensystem und die Koordinatentransformation, die das erste Bild dem zweiten Bild zuordnet (oder andersherum) können nach einem weiteren erfindungsgemäßen Aspekt mit den Bilddaten des zweiten Bildes für weitere (nachfolgende) Auswerte- und/oder Bearbeitungsschritte verwendet werden. Es kann somit in nachfolgenden Bearbeitungsschritten ein direkter Bezug zu Daten aus dem ersten Bild hergestellt werden.

In einer besonders vorteilhaften Ausgestaltung des erfindungsgemäßen Verfahrens wird dem ausgehärteten Präparatblock ein durchgehendes Koordinatensystem zugeordnet und es werden Transformationsvorschriften (Koordinatentransformationen) bestimmt, die entsprechende Bildabschnitte der ursprünglichen Probe geometrisch auf die eingebettete Probe abbilden. Die so definierten Koordinaten- und Transformationsvorschriften können auch in weitere Präparationsschritte übernommen werden. So können z.B. einer Stelle in einem fertigen TEM-Präparat der Probe mittels Transformation direkt Bilddaten aus dem ersten lichtmikroskopischen Verfahren (also der ursprünglichen Probe/dem Lebendpräparat) zugeordnet werden.

Das erfindungsgemäße Verfahren kann also insbesondere auch Teil eines größeren Probenbearbeitungs- und Bildaufnahmeprozesses sein, insbesondere eines Verfahrens zur Durchführung von korrelativer Licht- und Elektronenmikroskopie (Correlative light and electron microscopy, CLEM). Hier würde sich ein Schnittprozess (beispielsweise mittels eines Mikrotoms, vorzugsweise eines Ultramikrotoms) und eine Untersuchung mittels eines Elektronenmikroskops (beispielsweise eines Transmissions-Elektronenmikroskops, TEM) anschließen, nachdem der Präparatblock vom Probenträger zumindest teilweise gelöst wurde. Nach einem zumindest teilweisen Ablösen des ausgehärteten Einbettmediums mit der ausgehärteten Probe vom Probenträger können Probenschnitte durch Schneiden der Probe im die Probe einbettenden Einbettmedium (also durch Schneiden des Präparatblocks) erfolgen, wobei diese Probenschnitte danach in einem Elektronenmikroskop untersucht werden. Die im Rahmen des erfindungsgemäßen Verfahrens gewonnenen Transformationsvorschriften können einschließlich der definierten Koordinatensysteme an die anschließend verwendeten Geräte (wie beispielsweise ein Elektronenmikroskop) übermittelt und dort weiterverarbeitet und/oder bei der Durchführung weiterer Prozessschritte berücksichtigt werden.

Noch einmal zusammengefasst ist ein wichtiger Aspekt der Erfindung das Aufnehmen eines zweiten lichtmikroskopischen Bildes der eingebetteten Probe durchzuführen, noch während sie in festem Kontakt mit dem verwendeten Probenträger steht, und diese Aufnahme vorteilhafterweise auch mit so durchzuführen, dass die Auflösung des zweiten Bildes der des ersten Bildes möglichst ähnlich ist, und zwar durch Abbilden des gesamten oder zumindest eines großen Teils des Präparats. Dieses zweite Bild sollte dann durch den Benutzer oder auch durch eine elektronische Korrelationsroutine zum ersten Bild orientiert und die Bildbereiche zugeordnet werden. Alternativ ist natürlich auch eine umgekehrte Transformation möglich: Das erste Bild kann ebenfalls neu orientiert und die Bildbereiche den Bereichen des zweiten Bildes zugeordnet werden. Die Aufnahme des zweiten Bilds erfolgt insbesondere durch den Probenträger hindurch.

Durch dieses Vorgehen ist eine genaue Zuordnung zwischen Probenbereichen im Präparatblock bzw. dem zweiten Bild und den gleichen Probenbereichen in der ursprünglichen Probe bzw. dem ersten Bild erreichbar. Sollte durch das Ablösen des Trägermaterials eine Beschädigung der Blockoberfläche erfolgen, oder die Gitter- oder Netzstruktur ganz oder teilweise verloren gehen, gibt es mit den Scans des unbeschädigten Blocks die bestmöglichen Daten zur Relokalisation einzelner interessanter Probenbereiche. Diese Relokalisation kann durch Aufsuchen von Koordinatenpunkten oder durch lokale Korrelation des Scans mit unbeschädigten Probenbereichen erfolgen. Sie eröffnet auch die Möglichkeit automatisierter Weiterbearbeitung.

Durch das erfindungsgemäße Verfahren wird eine im Vergleich zum Stand der Technik wesentlich höhere Erfolgsrate im Aufsuchen interessierender Probenbereiche wie Zellstrukturen erreicht. Das gilt sowohl im Fall des lichtmikroskopisch-manuellen Aufsuchens/Vergleichens der beiden lichtmikroskopischen Bilder als auch für eine automatische Korrelation der Bilddaten mittels eines Algorithmus. Wenn auch nur kleine Probenbereiche nach Ablösen des Probenträgers unbeschädigt bleiben, können sie eindeutig identifiziert und so optimal ausgenützt werden. Selbst wenn der Probenträger in den Schritten vor der Polymerisation (z.B. während des Hochdruckgefrierens) bricht, können (größere) Splitter des Probenträgers mit der Probe nach der Polymerisation erfolgreich zugeordnet werden und sind so nicht für geplante Untersuchungen verloren.

Es gibt nun verschiedene Möglichkeiten, die Lehre der vorliegenden Erfindung in vorteilhafter Weise auszugestalten und weiterzubilden. Dazu ist einerseits auf die dem Patentanspruch 1 nachgeordneten Patentansprüche und andererseits auf die nachfolgende Erläuterung der Erfindung anhand der Zeichnungen zu verweisen. In Verbindung mit der Erläuterung der bevorzugten Ausführungsbeispiele der Erfindung anhand der Zeichnungen werden auch im Allgemeinen bevorzugte Ausgestaltungen und Weiterbildungen der Lehre erläutert.

Der Begriff "und/oder" umfasst alle Kombinationen von einem oder mehreren der zugehörigen aufgeführten Elemente und kann mit "/" abgekürzt werden.

Obwohl einige Aspekte im Rahmen einer Vorrichtung beschrieben wurden, ist es klar, dass diese Aspekte auch eine Beschreibung des entsprechenden Verfahrens darstellen, wobei ein Block oder eine Vorrichtung einem Verfahrensschritt oder einer Funktion eines Verfahrensschritts entspricht. Analog dazu stellen Aspekte, die im Rahmen eines Verfahrensschritts beschrieben werden, auch eine Beschreibung eines entsprechenden Blocks oder Elements oder einer Eigenschaft einer entsprechenden Vorrichtung dar.

### Kurze Beschreibung der Zeichnungen

Figuren 1a und 1b zeigen Varianten von Kryoröhrchen.
Figur 2a bis 2c zeigen verschiedene lichtmikroskopische Bilder, die im Rahmen eines Verfahrens gemäß Ausgestaltungen der Erfindung aufgenommen worden sind.
Figur 3 zeigt eine Überlagerung zweier Bilder, die im Rahmen eines Verfahrens gemäß einer Ausgestaltung der Erfindung aufgenommen worden sind.
Figur 4 zeigt ein Flussdiagramm zur Verdeutlichung des erfindungsgemäßen Verfahrens.
Figur 5 veranschaulicht ein Mikroskopsystem, das zur Durchführung eines Verfahrens gemäß einer Ausgestaltung der Erfindung verwendet werden kann.
Figur 6 veranschaulicht mehrere erfindungsmäße Möglichkeiten zur Beleuchtung eines Präparatblocks zur Aufnahme eines Bildes.
Figur 7 zeigt eine weitere erfindungsgemäße Möglichkeit zur Beleuchtung eines Präparatblocks zur Aufnahme eines Bildes.

In den Figuren sind identische oder baulich oder funktional einander entsprechende Komponenten mit identischen Bezugszeichen angegeben und werden der Übersichtlichkeit nicht wiederholt erläutert. Erläuterungen bezüglich Vorrichtungsmerkmalen oder entsprechenden Komponenten betreffen auch entsprechend durchgeführte Verfahrensschritte und umgekehrt.

### Ausführliche Beschreibung der Zeichnungen

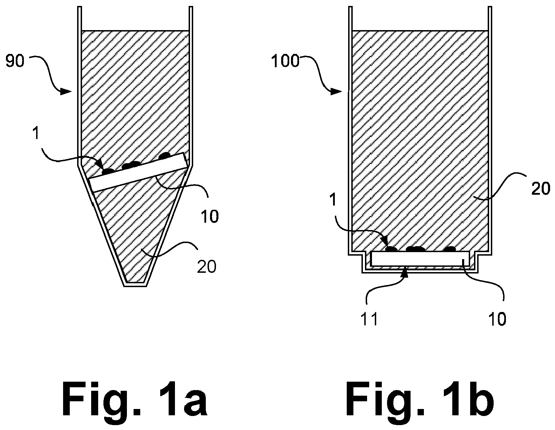

Die Figuren 1a und 1b zeigen Varianten von Kryoröhrchen in einer stark vereinfachten und rein schematischen Darstellung. Im Detail zeigt die Figur 1a ein Kryoröhrchen 90 mit einem konischen Boden. In diesem Fall liegt der Probenträger, wie in Fig. 1a gezeigt und mit 10 bezeichnet, oft nicht senkrecht zur Längsachse des Kryoröhrchens 90 und hat auf beide Seiten massive Kunstharzblöcke eines schraffiert veranschaulichten Einbettmediums 20 anpolymerisiert, die nicht einfach abzulösen sind. Präparatträgersplitter sind in diesem Fall typischerweise einpolymerisiert und meistens nur mit großem Aufwand zu befreien. Eine auf dem Probenträger 10 angeordnete Probe ist mit 1 bezeichnet.

In einer bevorzugten Variante des erfindungsgemäßen Verfahrens werden die Präparate mittels Flow-Through-Ringen/Einsätzen oder ähnlich konstruierter Kryoröhrchen mit planarem und ausreichend großen Boden ausgehärtet bzw. polymerisiert. Eine solche Art von Kyroröhrchen (ein Flow-Through-Einsatz) ist in Figur1b gezeigt und mit 100 bezeichnet, wobei die Figur 1b nur rein schematisch einen Flow-Through-Ring zeigt, aber auch ggf. eine von mehreren Kammern (Abschnitten) zur Probenaufnahme eines Flow-Through-Einsatzes. Diese Kammern haben einen Durchmesser von 6 mm zur Aufnahme eines entsprechend großen Probenträgers bzw. Saphirs. Ein solcher Flow-Through-Einsatz kann nach außen berandet oder nicht berandet sein. In letzterem Fall muss er in ein umgebendes Behältnis wie eine Wanne eingebracht werden, bevor das Einbettmedium 20 hinzugefügt wird.

Wie in Figur 1b sichtbar ist, liegen die Probenträger 10 im Fall der Verwendung solcher Kryoröhrchen auf einer ebenen Fläche auf, auf der Rückseite 11 des Probenträgers 10 entsteht nur eine sehr dünne Schicht aus dem Einbettungsmedium 20, also beispielsweise dem Kunstharz. Der verwendete Flow-Through-Ring kann folglich von dieser Kunstharzschicht gut abgelöst werden und die dünne Kunstharzschicht kann relativ einfach mechanisch vom glatten Trägermaterial entfernt werden, z.B. mit einem Skalpell. Diese Methode kann auch für Probenträgersplitter (Teile eines Probenträgers) angewendet werden. Im Gegensatz dazu liegt der Probenträger in einem Kryoröhrchen wie es beispielsweise in Figur 1a dargestellt ist, oft nicht senkrecht zur Achse und hat auf beide Seiten massive Kunstharzblöcke anpolymerisiert, die nicht einfach abzulösen sind. Splitter des Probenträgers 10 sind in diesem Fall typischerweise einpolymerisiert und meistens nur mit großem Aufwand zu befreien.

Nach Auslösen des Kunstharzblocks aus der Polymerisationsform 90, 100 (also einem Kryröhrchen) muss die Rückseite des Trägerplättchens bzw. Probenträgers 10 zuerst vom Einbettmedium 20, beispielsweise dem Kunstharz, befreit werden. Im Fall von Saphir als Trägermaterial und beispielsweise Epoxidharz als Kunstharz kann dieses Entfernen sehr effizient auf mechanischem Weg mit einem Skalpell erfolgen. Auch die Verwendung geeigneter Lösungsmittel ist möglich.

Wenn die Rückseite des Präparats bzw. Probenträgers 10 gut gesäubert ist (abschließend kann hier auch ein Waschen mit z.B. Ethanol erfolgen), wird es in eine Halterung aufgenommen und auf dem Mikroskoptisch platziert. Hier kann es nun lichtmikroskopisch untersucht werden, wobei idealerweise das gleiche Objektiv oder sogar dasselbe Mikroskop verwendet wird (oder ein Mikroskop desselben Typs), da ja auch das gleiche Trägermaterial durchstrahlt wird. Damit ist gewährleistet, dass beispielsweise die Vergrößerung und Auflösung der Bilder möglichst identisch sind. Es kann aber auch ein anderes Objektiv verwendet werden, die daraus resultierende Effekte sind dann bei der Datenauswertung zu berücksichtigen, bspw. die unterschiedliche Vergrößerung.

Als lichtmikroskopische Methode kann Durchlichtmikroskopie verwendet werden. Wenn die Vorpräparation die Präparatfluoreszenz nicht zerstört hat, ist auch der Einsatz von Fluoreszenzmikroskopie denkbar. Wenn das Kunstharz selbst fluoresziert (Autofluoreszenz, wie z.B. im Fall von Epoxidharz) kann dieses Fluoreszenzlicht als Durchlichtquelle für das Präparat verwendet werden.

Vorteilhaft wird nun das ausgehärtete/einpolymerisierte Präparat mit Trägermaterial auf die gleiche Art, jedenfalls aber an den gleichen Stellen, abgescannt wie das Lebendpräparat. Auch diese Scans werden zu Gesamtbildern vereinigt und können (bei Verwendung des gleichen Objektivs) im Wesentlichen durch Drehung mit den Lebendzellaufnahmen zur Deckung gebracht werden. Wenn ein anderes Objektiv verwendet wird, muss auch die Vergrößerung einer der beiden Bildgruppen angepasst werden. Die Untersuchung und Bildaufnahme erfolgt vorteilhafterweise durch den Probenträger hindurch.

Die Bilder können nun zur Deckung gebracht werden und ein Benutzer kann z.B. durch teiltransparente Überlagerung einzelne Zellen oder Zellgruppen identifizieren und ihre Lage für die weitergehende Präparation aus den Bildern vermessen. Genauso gut kann diese Anpassung aber auch maschinenbasiert automatisch erfolgen, wobei idealerweise gleichzeitig Koordinaten erfasst (semiautomatisch oder automatisch) und abgespeichert werden.

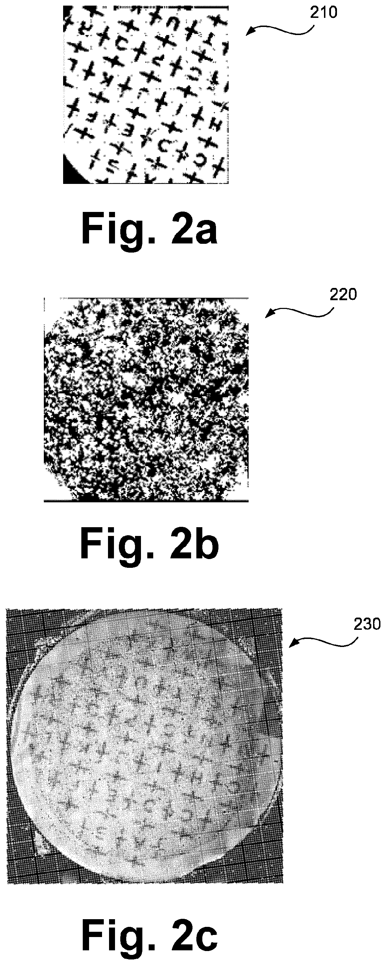

Die beiden Figuren 2a und 2b zeigen zusammengesetzte Bilder 210 und 220 eines untersuchten Lebendpräparats in Durchlicht- und Fluoreszenzabbildung. Die Bilder 210 und 220 wurden im dargestellten Beispiel mit einem 40fach-Objektiv erhalten. Bild 210 wurde mittels Durchlichtmikroskopie erhalten. Gemäß Bild 220 wurden im Präparat gebundene Fluoreszenzmarker mit einer Wellenlänge von 470 nm angeregt und bei einer Wellenlänge von 510 nm detektiert. Dies sind Beispiele für Bilder aus dem ersten lichtmikroskopischen Verfahren. Die Figur 2c zeigt eine zusammengesetzte Aufnahme 230 (das zweite Bild) der Probe bzw. des Präparatblocks durch den Trägersaphir (also den Probenträger). Es handelt sich um eine Durchlicht-Hellfeldaufnahme durch ein 20fach-Objektiv. Deutlich sichtbar ist hier jeweils die Netz-/Gitterstruktur auf dem Probenträger, die der Orientierung dienen kann.

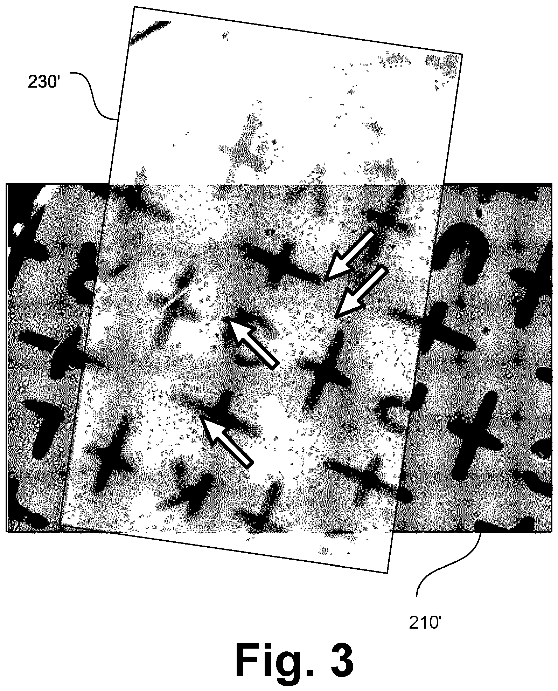

Wie in Figur 3 gezeigt, können diese Abbildungen, hier mit 210' und 230' bezeichnet, nun z.B. zur Auswertung durch den Anwender teiltransparent überlagert werden: Die Pfeile zeigen beispielsweise korrelierte interessante Zellformationen

Alternativ kann eine solche Identifizierung sich entsprechender Bildausschnitte auch softwarebasiert durch einen Algorithmus erfolgen (Korrelations-Software): Diese Korrelationssoftware wertet die ersten und zweiten Bilddaten aus und bestimmt anhand von sich entsprechenden Bereichen eine Koordinatentransformation zwischen dem ersten und dem zweiten Bild. Hierdurch können die Koordinaten von ursprünglich interessierenden Probenbereichen direkt im Präparatblock bestimmt und für weitere Prozessschritte, die manuell oder automatisiert erfolgen können, zur Verfügung gestellt werden. Ein solcher Korrelationsalgorithmus kann auch Machine/Deep-Learning-basiert sein.

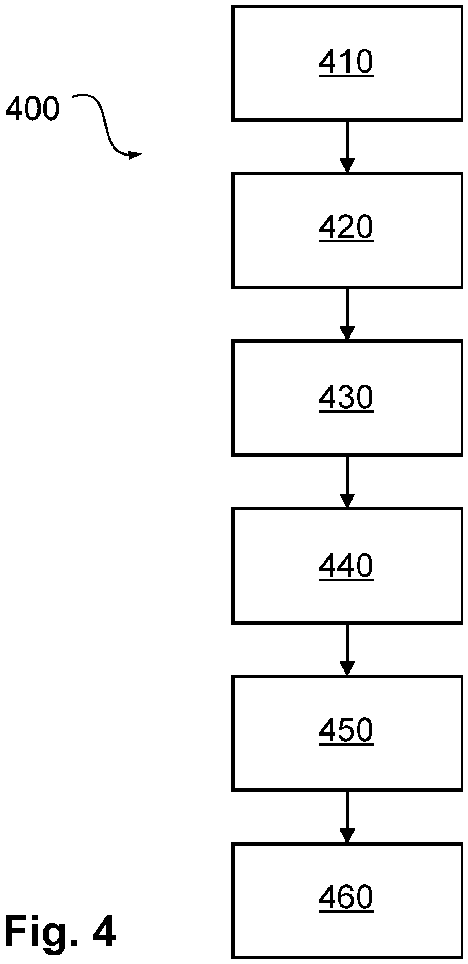

Figur 4 schließlich zeigt als Verlaufsdiagramm die einzelnen Verfahrensschritte eines Verfahrens 400 gemäß einer beispielhaften Ausgestaltung der Erfindung: Eine biologische Probe wird in einem ersten Schritt 410 auf einen vorzugsweise transparenten Probenträger aufgebracht, beispielsweise aufgewachsen. Nach anschließender Aufnahme eines ersten lichtmikroskopischen Bildes, beispielsweise eines Durchlichtbildes oder eines fluoreszenzmikroskopischen Bildes der Probe, in Schritt 420 erfolgt in einem weiteren Schritten 430 ein Kryofixieren, Gefriersubstituieren und anschließendes Infiltrieren und Einbetten der Probe mit einem Einbettmedium, wie beispielsweise einem Kunstharz. Dieses Einbettmedium wird nun in Schritt 440 ausgehärtet. Anschließend werden der Präparatblock und der Probenträger aus der Einbettform entnommen. Dann erfolgt (nach einem optionalen Ablösen bzw. Entfernen des ausgehärteten Einbettmediums von der Rückseite des Probenträgers) im nachfolgenden Schritt 450 ein weiteres lichtmikroskopisches Abbilden der Probe, insbesondere durch den Probenträger hindurch. Die Bilder aus den Schritten 420 und 450 werden nun miteinander verglichen und sich entsprechende Bildausschnitte bzw. Probenbereiche identifiziert. Dieser Schritt kann entweder durch einen Nutzer erfolgen oder auch automatisiert, beispielsweise durch Machine/Deep-Learning-gestützte Bildererkennungsverfahren. Hierdurch wird im letzten Schritt 460 eine Koordinatentransformation zwischen den beiden Bildern bzw. Bilddaten bestimmt. Diese kann benutzt werden, um nach der Abtrennung des Probenträgers vom Präparatblock, auch wenn hierdurch Teile der Probe zerstört werden sollten, Bildbereiche aus dem zweiten Bild eindeutig Bildebereichen aus dem ersten Bild zuzuordnen. Anders ausgedrückt kann die Lage von im ersten Bild identifizierten Probenbereichen nach dem Einfrieren und Einbetten der Probe auch im zweiten Bild und auch im elektronenmikroskopischen Bild einfach bestimmt werden.

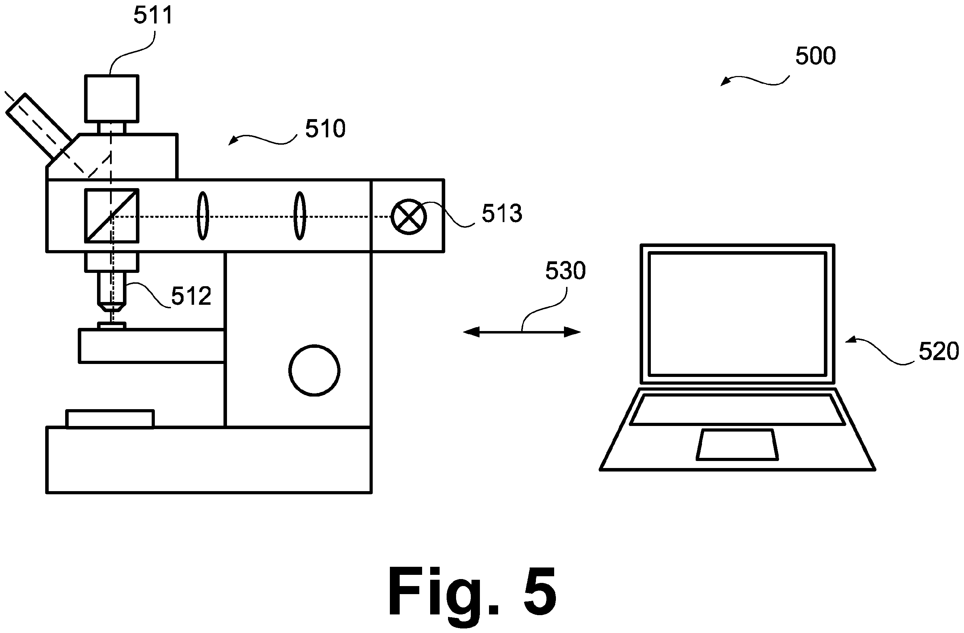

In Figur 5 ist ein Mikroskopsystem 500 dargestellt, das zur Durchführung eines Verfahrens gemäß einer Ausgestaltung der Erfindung eingerichtet sein kann, und das ein (Fluoreszenz-) Mikroskop 510 und ein Computersystem 520 aufweisen kann, die drahtgebunden oder drahtlos miteinander kommunizieren können, wie mit einem Doppelpfeil 530 veranschaulicht. Bei dem Mikroskop 510 kann es sich um ein aufrechtes (wie dargestellt), aber alternativ auch um ein inverses Mikroskop handeln.

Das Computersystem 520 kann ausgebildet sein zum Ausführen eines Maschinenlern-Algorithmus. Das Computersystem 520 und das Mikroskop 510 können getrennte Einheiten sein, können aber auch zusammen in einem gemeinsamen Gehäuse integriert sein. Das Computersystem 520 kann Teil eines zentralen Verarbeitungssystems des Mikroskops 510 sein und/oder das Computersystem 520 kann Teil einer Teilkomponente des Mikroskops 510 sein, wie eines Sensors, eines Aktuators, einer Kameraeinheit 511 oder einer Beleuchtungseinheit, usw. des Mikroskops 510.

Einige oder Verfahrensschritte, die hierfür geeignet sind, können durch (oder unter Verwendung) einer Hardwarevorrichtung ausgeführt werden, wie es zum Beispiel ein Prozessor, ein Mikroprozessor, ein programmierbarer Computer oder eine elektronische Schaltung sein kann. In einigen Ausführungsbeispielen können ein oder mehrere der wichtigsten Verfahrensschritte durch eine solche Vorrichtung ausgeführt werden.

Insbesondere kann das zuvor mit 420 bezeichnete Aufnehmen eines ersten Bildes einer auf einem Probenträger aufgebrachten Probe mit einem ersten lichtmikroskopischen Verfahren unter Verwendung der Kameraeinheit 511 des Mikroskops 510 erfolgen, ebenso wie das zuvor mit 450 bezeichnete Aufnehmen eines zweiten Bildes der im ausgehärteten Einbettmedium eingebetteten Probe mit einem zweiten lichtmikroskopischen Verfahren. Insbesondere kann das Aufnehmen 420 des ersten Bildes und das Aufnehmen 450 des zweiten Bildes unter Verwendung desselben Mikroskops 510 erfolgen, und weiter insbesondere unter Verwendung desselben Objektivs 512 des Mikroskops 510 und/oder derselben Vergrößerung und/oder desselben Scanbereichs und/oder derselben Bildauflösung.

Das Mikroskop 510 kann zur Auflicht- oder Durchlichtbeleuchtung eingerichtet sein und das Verfahren kann insbesondere umfassen, dass zur erfindungsmäßen Aufnahme eines zweiten Bildes 230 eine Autofluoreszenz des Einbettmediums dadurch angeregt wird, dass das Einbettmedium durch eine künstliche Lichtquelle 513, beispielsweise eine LED oder einen Laser, beleuchtet wird.

Insbesondere kann die Zuordnung sich entsprechender Probenbereiche auf Basis der ersten Bilddaten und der zweiten Bilddaten durch einen ersten Algorithmus erfolgen, der in dem Computersystem 520 ausgeführt wird. Einem ersten Bild kann dabei insbesondere ein erstes Koordinatensystem und einem zweiten Bild ein zweites Koordinatensystem zugeordnet werden, und ausgehend von den miteinander identifizierten Bildbereichen kann eine Koordinatentransformation zwischen dem ersten Koordinatensystem und dem zweiten Koordinatensystem durch den ersten oder einen zweiten Algorithmus bestimmt werden. Das zweite Koordinatensystem und die Koordinatentransformation mit den Bilddaten des zweiten Bildes können für weitere Auswerte- und/oder Bearbeitungsschritte verwendet und dabei beispielsweise in dem Computersystem 520 zwischengespeichert werden.

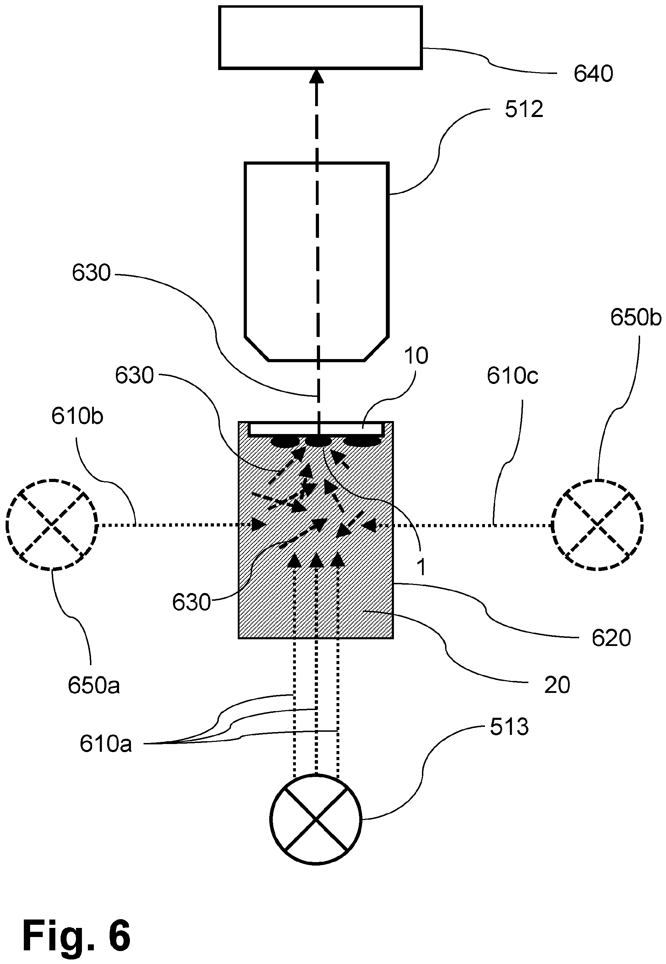

Figur 6 zeigt nun eine schematisch erfindungsmäße Möglichkeiten zur Beleuchtung einer Probe 1 in einem Einbettmedium 20 und der Aufnahme eines zweiten Bildes 230: Mittels einer künstlichen Lichtquelle 513, beispielsweise einem Laser oder einer LED, wird Licht 610a in den Präparatblock 620 mit der Probe 1 eingestrahlt. Hierbei gibt es mehrere Möglichkeiten zur Beleuchtung der Probe 1: Das Licht 610a dient entweder selbst der Durchlichtbeleuchtung der Probe 1 und/oder regt im Einbettmedium 20 Autofluoreszenz 630 an (das dann zur Durchlichtbeleuchtung der Probe 1 dient) und/oder regt in der Probe 1 Fluoreszenz an (falls die Probe geeignete Fluorophore beinhaltet). Das durch den Probenträger 10 transmittierte Licht 630 wird nun mittels eines Objektives 512 aufgefangen und zu einem Detektor 640, beispielsweise einer Kamera oder einem Punktdetektor, geleitetet. Die in Fig. 6 veranschaulichten Varianten zeigen eine Detektion durch den Probenträger 10 hindurch, dies muss aber nicht zwingend der Fall sein, eine Detektion kann auch aus anderen Richtungen erfolgen, Fig. 6 zeigt hier nur eine besonders vorteilhafte Ausgestaltung der Erfindung. Ebenso muss die Beleuchtung des Präparatblocks 20 nicht zwingend aus der gezeigten Richtung 610a (also von der dem Probenträger 10 und dem Objektiv 512 abgewandten Seite des Präparatblocks 620) erfolgen. Alternativ oder auch zusätzlich ist eine Beleuchtung auch aus anderen Richtungen 610b, 610c denkbar, wofür die Lichtquelle 513 entsprechend platziert oder eine oder mehrere zusätzliche Lichtquellen 650a, 650b eingesetzt werden können. Auch das Aufspalten und/oder Auffächern von Licht einer Lichtquelle 513 ist denkbar, um ein Einstrahlen von Licht aus verschiedenen Richtungen auf den Präparatblock 20 zu ermöglichen und eine besonders vorteilhafte Anregung von Autofluoreszenz zu gewährleisten.

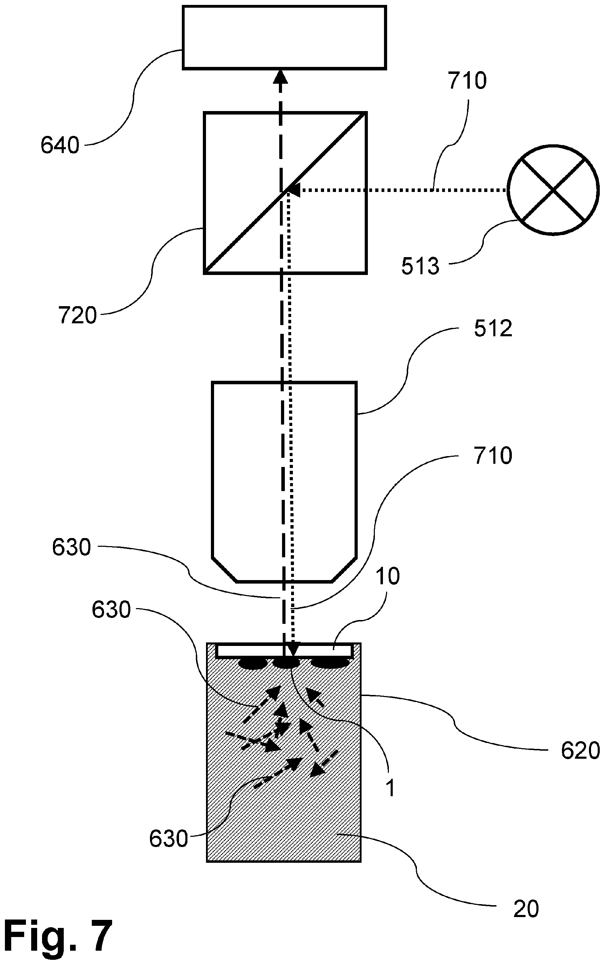

Besonders vorteilhaft ist es, wenn die Beleuchtung durch den Probenträger 10 erfolgt, wobei die künstliche Lichtquelle 513 dazu dient, eine Fluoreszenz in der Probe 1 und/oder eine Autofluoreszenz im Einbettmedium 20 anzuregen. Diese Möglichkeit der Ausgestaltung ist in Figur 7 schematisch gezeigt. Hierbei kann eine geeignete Lenkung des Lichts 710, das der Beleuchtung der Probe bzw. des Einbettmediums 20 dient, und des zu detektierenden Lichts 630 mittels eines Strahlteilers 720, wobei es sich vorzugsweise um einen dichroitischen Strahlteiler handelt, realisiert sein.

## Patentansprüche

1. Verfahren (400) zur mikroskopischen Untersuchung einer Probe (1), umfassend die folgenden Schritte:
a. Aufbringen (410) der Probe (1) auf einen Probenträger (10) mit oder bestehend aus einem lichtdurchlässigen Trägermaterial,
b. Aufnehmen (420) eines ersten Bildes (210, 220) der auf dem Probenträger (10) aufgebrachten Probe (1) mit einem ersten lichtmikroskopischen Verfahren, wobei das erste Bild (210, 220) durch erste Bilddaten repräsentiert wird,
c. Kryofixieren, Gefriersubstituieren und anschließendes Infiltrieren und Einbetten (430) der Probe (1) zusammen mit dem Probenträger (10) mit einem Einbettmedium (20) in einer Einbettform (90, 100),
d. Aushärten (440) des Einbettmediums (20),
e. Entnahme der im ausgehärteten Einbettmedium (20) eingebetteten Probe (1) zusammen mit dem Einbettmedium (20) und dem Probenträger (10) aus der Einbettform (90, 100),
f. Aufnehmen (450) eines zweiten Bildes (230) der im ausgehärtetem Einbettmedium (20) eingebetteten Probe (1) mit einem zweiten lichtmikroskopischen Verfahren, wobei das zweite Bild durch zweite Bilddaten repräsentiert wird und durch das erste und das zweite Aufnehmen zumindest teilweise identische Bereiche der Probe (1) aufgenommen werden und
g. Identifizieren (460) von zumindest eines Ausschnittes des ersten Bildes (210, 220) und eines Ausschnittes des zweiten Bildes (230), die identische Bereiche der Probe (1) zeigen.

2. Verfahren (400) nach Anspruch 1, wobei das Aufnehmen (450) des zweiten Bildes (230) durch den Probenträger (10) hindurch erfolgt.

3. Verfahren (400) nach einem der vorherigen Ansprüche, wobei das Aufnehmen (420) des ersten Bildes (210, 220) und das Aufnehmen des zweiten Bildes (230) unter Verwendung desselben Mikroskops (510) erfolgen.

4. Verfahren (400) nach einem der vorherigen Ansprüche, wobei das Aufnehmen (420) des ersten Bildes (210, 220) und das Aufnehmen (450) des zweiten Bildes (230) unter Verwendung desselben Objektivs (512) des Mikroskops (510) und/oder derselben Vergrößerung und/oder desselben Scanbereichs und/oder derselben Bildauflösung erfolgen.

5. Verfahren (400) nach einem der vorherigen Ansprüche, wobei das Trägermaterial ein Glas oder Saphir ist.

6. Verfahren (400) nach einem der vorherigen Ansprüche, wobei die Probe (1) während der Aufnahme des ersten Bildes (210, 220) unter kontrollierten Umgebungsbedingungen gehalten wird.

7. Verfahren (400) nach einem der vorherigen Ansprüche, wobei das Einbettmedium (20) von der der Probe (1) abgewandten Seite des Probenträgers (10) vor dem Aufnehmen des zweiten Bildes (230) entfernt wird, wobei dieses Entfernen vorzugsweise auf mechanische Weise erfolgt.

8. Verfahren (400) nach einem der vorherigen Ansprüche, wobei das lichtmikroskopische Verfahren für die Aufnahme des zweiten Bildes Durchlicht-Hellfeldmikroskopie ist.

9. Verfahren (400) nach einem der vorherigen Ansprüche, wobei das Einbettmedium (20) Autofluoreszenz aufweist und das zweite lichtmikroskopische Verfahren die Autofluoreszenz zur Beleuchtung der Probe ausnutzt.

10. Verfahren (400) nach einem der vorherigen Ansprüche, wobei das Infiltrieren und Einbetten der Probe (1) in ein Einbettmedium (20) die folgenden Schritte umfasst:
a. Einbringen des Probenträgers (10) mit der Probe (1) in einem Kryoröhrchen (100) mit flachem Boden, wobei die maximale Ausdehnung des Bodens größer oder gleich ist als die maximale Ausdehnung des Probenträgers (10) und
b. Zuführen des Einbettmediums (20) in das Kryoröhrchen (100) mit der Probe (1), mindestens so lange bis die Probe (1) vollständig vom Einbettmedium (20) umschlossen ist, wobei der Probenträger (10) auch nach Zuführung des Einbettmediums parallel oder im Wesentlichen parallel zum Boden des Kryoröhrchens (100) orientiert ist.

11. Verfahren (400) nach einem der vorherigen Ansprüche, wobei sich entsprechende Probenbereiche auf Basis der ersten Bilddaten und der zweiten Bilddaten identifiziert werden, wobei diese Identifizierung durch einen ersten Algorithmus und/oder manuell erfolgt.

12. Verfahren (400) nach Anspruch 11, wobei dem ersten Bild ein erstes Koordinatensystem und dem zweiten Bild ein zweites Koordinatensystem zugeordnet ist und ausgehend von den miteinander identifizierten Bildbereichen eine Koordinatentransformation zwischen dem ersten Koordinatensystem und dem zweiten Koordinatensystem durch den ersten oder einen zweiten Algorithmus bestimmt wird.

13. Verfahren (400) nach Anspruch 12, wobei die Koordinatentransformation abschnittsweise bestimmt wird, so dass für unterschiedliche Probenbereiche und/oder unterschiedliche Zelltypen innerhalb der Probe unterschiedliche Koordinatentransformationen festgelegt werden.

14. Verfahren (400) nach Anspruch 12 oder 13, wobei das zweite Koordinatensystem und die Koordinatentransformation mit den Bilddaten des zweiten Bildes für weitere Auswerte- und/oder Bearbeitungsschritte verwendet werden.

15. Verfahren (400) nach einem der vorherigen Ansprüche, wobei das Verfahren weiterhin die folgenden Schritte umfasst:
a. Zumindest teilweise Ablösen des ausgehärteten Einbettmediums (20) mit der ausgehärteten Probe (1) vom Probenträger (10),
b. Herstellung eines Probenschnittes durch Schneiden der Probe im die Probe (1) einbettenden Einbettmedium (20), beispielsweise mit einem Mikrotom, und
c. Untersuchung des Probenschnittes in einem Elektronenmikroskop.
